Europäisches Patentamt

European Patent Office

Office européen des brevets

(11) Publication number: **0 251 430 B1**

# EUROPEAN PATENT SPECIFICATION

(45) Date of publication of patent specification: **09.10.91**   (51) Int. Cl.⁵: **A61H 23/00, H04R 1/00**

(21) Application number: **87302694.2**

(22) Date of filing: **27.03.87**

(54) **Vibrostimulative device.**

(30) Priority: **01.04.86 JP 72435/86**

(43) Date of publication of application:
   **07.01.88 Bulletin 88/01**

(45) Publication of the grant of the patent:
   **09.10.91 Bulletin 91/41**

(84) Designated Contracting States:
   **DE FR GB**

(56) References cited:
   **DE-A- 2 356 481**
   **DE-C- 821 271**
   **DE-C- 833 423**
   **DE-C- 859 054**
   **US-A- 2 920 617**

(73) Proprietor: **Hayashibara, Ken**
   **9-8, 4-chome, Higashi-Furumatsu**
   **Okayama-shi Okayama(JP)**

(72) Inventor: **Endo, Yoichi**
   **1-15, 5-chome, Jingumae Shibuya-ku**
   **Tokyo(JP)**

(74) Representative: **Palmer, Roger et al**
   **PAGE, WHITE & FARRER 54 Doughty Street**
   **London WC1N 2LS(GB)**

## Description

The present invention relates to a vibrostimulative device for example, a device to stimulate the human body with a vibration of a prescribed frequency.

Japanese Patent Laid-Open No. 209,355/83 discloses a vibrostimulative device wherein a vibratory plate to be placed on the human body is coupled through a closed gas space with a loudspeaker, while the voice coil of the loudspeaker is connected to a variable oscillator through an amplifier. The device has the advantages that its vibration frequency can be conveniently set to the most effective level for the subject; and that the gas used as the vibration-transmitting media transmits the vibration over a relatively large body area of the subject.

The device, however, has the drawback that, when the closed gas space is extended with a flexible hose having a relatively small diameter, it requires a relatively high power to attain a prescribed sound pressure on the vibratory plate because gases are generally low in vibration transmittability. This requires both the vibratory plate and the amplifier to be enlarged and the production cost of the device, is increased.

DE-A-2356481 describes a health developing device which may be used as a vibrostimulative device. The health developing device comprises a loud speaker connected in turn to an amplifier and a record player or the like. The loud speaker is situated within a closed gas space, one portion of the member which defines the closed gas space being a vibratory member on which a user may sit or lie. When in use, sound waves generated by the loudspeaker are transmitted through the closed gas space and cause vibration of the vibratory member against the user. In one embodiment the closed gas space is in the form of a mattress which may be inflated by either water or air.

An object of the present invention is to provide a vibrostimulative device wherein the drawbacks of the conventional device are reduced.

According to the present invention there is provided a vibrostimulative device comprising: an oscillator; an amplifier; a vibration generator connected with the oscillator through the amplifier; a vibratory member for transmitting vibration to a user; and a closed gas space for transmitting vibration from the vibration generator to the vibratory member, the gas space having an inlet for connection to compressing means characterised in that the closed gas space comprises a two part chamber in which the vibration generator is located, a first part of the chamber being forward of the vibration generator and a second part rearward of the vibration generator, the two parts being in communication by means of a narrow duct.

Preferred embodiments of the invention are now given by way of example only, with reference to the accompanying drawings in which:

FIG. 1 is a sectional side elevation view of an embodiment according to the invention;

FIG. 2 is a sectional side elevation view of another embodiment according to the invention; and

FIG. 3 is a sectional side elevation view of still another embodiment according to the invention.

In the drawings, reference numerals 1 and 12 designate box members; 2, is a loudspeaker; 3 is a cone; 4 is a vibratory member; 5 is an amplifier; 6 is an oscillator; 7 is a compressing means; 8 is a pressure gauge; 9 is a duct; 10 is a compressed closed gas space; 11 is a resilient hollow member; 13 is a resilient bag member; and 14 is a one-way restrictor valve.

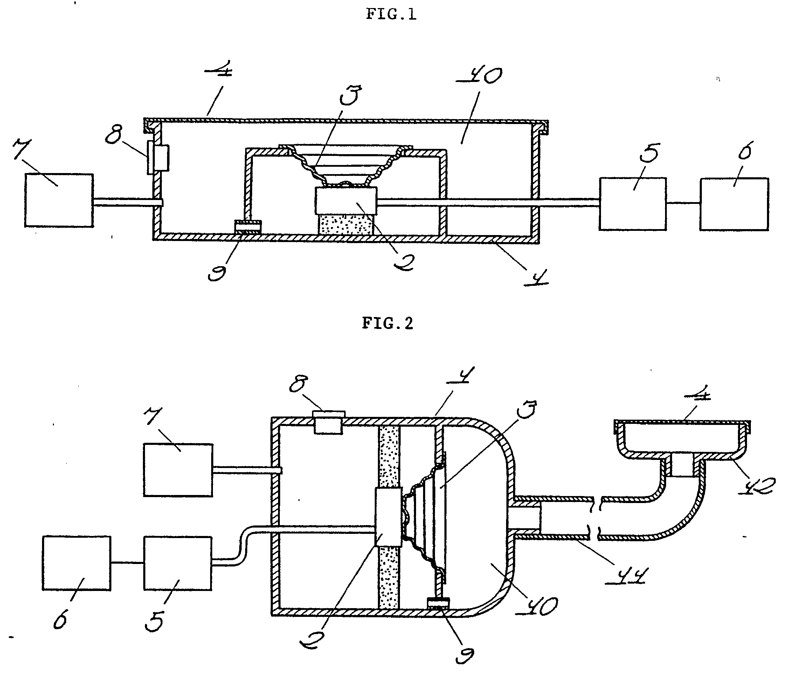

In the embodiment as shown in FIG. 1, a vibration generator comprising a loudspeaker (2) is placed upwardly at the center of the inside bottom surface of a first box member (1) which is capable of supporting a human body. An opening of the box member (1) is closed by a resilient metal sheet which forms an airtight closure, and acts as a vibratory member (4). A variable oscillator (6) is connected to the voice coil of loudpseaker (2) through an amplifier (5). A compressing means (7) and a pressure gauge (8) are provided to retain the compression in the box member (1). To equalize the pressures on both sides of the cone (3), a duct (9) is provided which allows the gas to surround both sides of the cone (3). The duct (9) should be of a size that does not create a substantial loss of vibration from the loudspeaker (2). Compression of a closed gas space (l0) formed between the loudspeaker (2) and the vibratory member (4) is generally attained by operating compressing means such as compressor or pump in a continuous or continual manner.

In this arrangement, a variable oscillator (6) is connected to a power source, and an adjusting dial (not illustrated) is then set to a prescribed frequency level. The output of the variable oscillator (6) is amplified by the amplifier (5), and supplied to the voice coil of loudspeaker (2), to vibrate the cone (3) at the prescribed frequency. The sound pressure produced is transmitted through the compressed closed gas space (l0), to vibrate the vibratory member (4). Thus, by placing the vibratory member (4) on the body of the subject, vibration treatment is carried out at the prescribed frequency. In this case, the frequency most effective for the subject is selected by first setting the device to the standard frequency, for example, 60 hertz, then gradually varying the frequency.

This embodiment can be favourably used in the form of a bed or chair to vibrate the whole body or a relatively large area of the body.

In the embodiment as shown in FIG. 2, the loudspeaker (2) which acts as the vibration generator is placed at the center of the inside bottom surface of a first box member (I), and one end of a resilient hollow member (II) having an airtight structure, such as flexible hose, is connected to the compressed closed gas space (I0) provided in front of the loudspeaker (2), through the wall of the box member (I). The other end of the resilient hollow member (II) is connected to a second box member (I2), which has an opening covered by a rubber sheet to form an airtight seal. The voice coil of the loudspeaker (2) is connected to a variable oscillator (6) through an amplifier (5), and the pressures at both sides of cone (3) are equalized through a duct (9). In order to efficiently transmit the vibration of the cone (3) to the vibratory member (4), the diameter of the cone (3) should be as close as possible to the diameter of resilient hollow member (II). The inside of the first box member (I) should be curved so that no undesirable resonance arises therein. Also, the pressure in the device should be increased to the highest possible level so that the vibratory member (4) extends to create a satisfactory cushioned surface. The second box member (I2) and the vibratory member (4) may be arranged in such a manner that the vibratory member (4) is shaped into a flat plate, tube or circle as disclosed, for example, in Japanese Patent Laid-Open No. 209,355/83, or Japanese Utility Model Laid-Open No. 52,827/85 or 52,828/85, and can be positioned on the affected site, whether the hands, feet, breast, or stomach.

This embodiment can be favourably used to effect vibration treatment by remote operation using an extended flexible hose. In this case, by providing two or more flexible hoses, two or more affected sites of one or more subjects are simultaneously administered with the vibration.

Although the previous embodiments are equipped with a compressing means, the present invention shall not be restricted to those. Any means can be employed as long as the closed gas space is compressed when in use: For example, the closed gas space can be compressed with compressing means which are then removed when in use. In this case, by ensuring that the device is sufficiently well air-sealed, the compression can be retained over a long period of time.

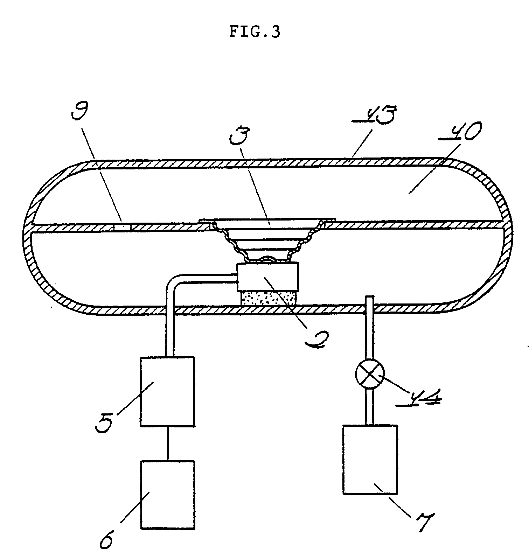

In the embodiment as shown in FIG. 3, compressing means can be omitted by airtightly enclosing the loudspeaker (2) in a resilient bag member (I3) resilient enough to support the body of the subject. The compression of the gas in the bag member (I3) is retained by shaping it into a cushion or mattress, and allowing the subject to lay thereon to press the bag member (I3). The compression in the bag member is controlled by providing a one-way restrictor valve (I4) in the wall of bag member (I3), to which compressing means are attached.

The vibratory member may be made with plastics material, rubber or metal as described above. The gas may be carbonated gas or helium gas, and shall not be restricted to air.

The vibration generator may be a loudspeaker or an electromagentic vibration generator, for example, as disclosed in Japanese Patent Laid-Open No. 209,284/85 or 2I6,870/85, where an air wave of compression and rarefaction is generated by continual compression of the air; and where an electric motor and a vibratory plate are used in combination.

In a vibrostimulative device, as described above, the frequency variable vibration generator and the vibratory member transmitting the vibration to the body of the subject are coupled through the compressed closed gas space, and hence the most effective vibration is efficiently transmitted locally or systematically to the subject. Thus, the present invention is characterized in that reduction or suppression of shoulder discomfort, myalgia, low back pain and asthmatic spasm, and improvement of blood circulation is attained by using less electricity than in the case of using a conventional device.

Furthermore, since the present invention reduces the loss of vibration in the closed gas space, a low-power amplifier and a relatively small vibration generating means can be advantageously used, similarly an extended flexible hose having a relatively small diameter may be used. Because of this, unlike the conventional devices, the device of the present invention is neither bulky nor does the hose interfere with treatment of the subject.

## Claims

1. A vibrostimulative device comprising:

an oscillator (6); an amplifier (5); a vibration generator (3) connected with the oscillator (6) through the amplifier (5); a vibratory member (4) for transmitting vibration to a user; and a closed gas space (10) for transmitting vibration from the vibration generator (3) to the vibratory member (4), the gas space (10) having an inlet for connection to compressing means (7) characterised in that the closed gas space comprises a two part chamber (10) in which the vibration generator (3) is located, a first part of the chamber (10) being forward of the vibration generator (3) and a second part rearward of the vibration generator (3), the two parts being in communication by means of a narrow duct (9).

2. A device according to Claim 1, wherein said vibration generator (3) is a loudspeaker.

3. A device according to Claim 1 or Claim 2, wherein said vibratory member (4) is made of rubber, metal or plastics material.

4. A device according to Claims 1, 2, or 3, which has a plurality of vibratory members (4).

5. A device according to any of the foregoing claims, which comprises:
   a first box member (1) enclosing the vibration generator (3);
   a hollow member (11); and
   a second box member (12) having an opening covered airtightly with the vibratory member (4), the second box member (12) being airtightly jointed to the first box member (1) through the hollow member (11).

6. A device according to claim 5, wherein said resilient hollow member (11) is a flexible hose.

7. A device according to any of the foregoing claims, which comprises:
   a resilient bag member (13) enclosing the vibration generator (3), the inside space of said bag member (13) being brought into compression when pressed, a part of said bag member (13) acting as a vibratory member (4) when actuated with the vibration generator (3).

8. A device according to claim 7, in which the bag member (13) is in the form of a cushion.

9. A device according to claim 7, in which the bag member (13) is in the form of a mattress.

10. A device according to any one of the foregoing claims, which further comprises compressing means (7).

11. A device according to any of the foregoing claims, wherein said compressed closed gas space (10) is filled with air.

**Revendications**

1. Dispositif de stimulation par vibrations comprenant :
   un oscillateur (6) ; un amplificateur (5) ; un générateur de vibrations (3) connecté à l'oscillateur (6) par l'intermédiaire de l'amplificateur (5) ; un élément vibrant (4) pour transmettre des vibrations à un utilisateur ; et un espace fermé rempli de gaz (10) pour transmettre des vibrations du générateur de vibrations (3) à l'élément vibrant (4), l'espace de gaz (10) comportant une entrée pour la connexion à des moyens de compression (7) caractérisé en ce que l'espace fermé de gaz comprend une chambre en deux parties (10) dans laquelle le générateur de vibrations (3) est placé, une première partie de la chambre (10) étant à l'avant du générateur de vibrations (3) et une seconde partie étant à l'arrière du générateur de vibrations (3), les deux parties communiquant par l'intermédiaire d'un conduit étroit (9).

2. Dispositif selon la revendication 1, dans lequel ledit générateur de vibrations (3) est un haut-parleur.

3. Dispositif selon la revendication 1 ou la revendication 2, dans lequel ledit élément vibrant (4) est constitué de caoutchouc, de métal ou de matière plastique.

4. Dispositif selon la revendication 1, 2 ou 3, qui comporte une pluralité d'éléments vibrants (4).

5. Dispositif selon l'une quelconque des revendications précédentes, qui comprend :
   un premier élément en forme de caisse (1) contenant le générateur de vibrations (3) ;
   un élément creux (11), et
   un second élément en forme de caisse (12) comportant une ouverture recouverte de manière étanche à l'air avec l'élément vibrant (4), le second élément en forme de caisse (12) étant relié de manière étanche à l'air au premier élément en forme de caisse (1) à travers l'élément creux (11).

6. Dispositif selon la revendication 5, dans lequel ledit élément creux élastique (11) est un tuyau flexible.

7. Dispositif selon l'une quelconque des revendications précédentes, qui comprend :
   un élément de sac élastique (13) contenant le générateur de vibrations (3), l'espace intérieur dudit élément de sac (13) étant amené en compression lorsqu'il est compressé, une partie dudit élément de sac (13) agissant comme un élément vibrant (4) quand elle est actionnée par le générateur de vibrations (3).

8. Dispositif selon la revendication 7, dans lequel l'élément de sac (13) a la forme d'un coussin.

9. Dispositif selon la revendication 7, dans lequel l'élément de sac (13) a la forme d'un matelas.

10. Dispositif selon l'une quelconque des revendications précédentes, qui comprend de plus des moyens de compression (7).

11. Dispositif selon l'une quelconque des revendications précédentes, dans lequel ledit espace fermé rempli de gaz comprimé (10) est rempli avec de l'air.

**Patentansprüche**

1. Vibrations-Stimulationsgerät, bestehend aus: einem Oszillator (6); einem Verstärker (5); einem Schwingungserzeuger (3), der mit dem Oszillator (6) über den Verstärker (5) verbunden ist; einem Schwingteil (4) zur Übertragung der Vibration auf einen Benützer; und aus einem geschlossenen Gasraum (10) mit einem Einlaß für den Anschluß an eine Druckeinrichtung (7), dadurch gekennzeichnet, daß der geschlossene Gasraum eine zweiteilige Kammer (10) umfaßt, in welcher der Schwingungserzeuger (3) untergebracht ist, wobei ein erster Teil der Kammer (10) vor dem Schwingungserzeuger (3) und ein zweiter Teil hinter dem Schwingungserzeuger (3) liegt und die beiden Teile miteinander durch einen engen Durchlaß (9) in Verbindung stehen.

2. Vorrichtung nach Anspruch 1, wobei der Schwingungserzeuger (3) ein Lautsprecher ist.

3. Vorrichtung nach Anspruch 1 oder 2, wobei der Schwingteil (4) aus Gummi, Metall oder Kunststoffmaterial besteht.

4. Vorrichtung nach Anspruch 1, 2 oder 3, welche eine Anzahl von Schwingteilen (4) anfweist.

5. Vorrichtung nach einem der vorangehenden Ansprüche, welche folgendes umfaßt: einen ersten Kastenteil (1), der den Schwingungserzeuger (3) umschließt; einen Hohlteil (11); und einen zweiten Kastenteil (12) mit einer Öffnung, welche luftdicht mit dem Schwingteil (4) abgedeckt ist, wobei der zweite Kastenteil (12) luftdicht mit dem ersten Kastenteil (1) durch den Hohlteil (11) verbunden ist.

6. Vorrichtung nach Anspruch 5, wobei der elastische Hohlteil (11) ein flexibler Schlauch ist.

7. Vorrichtung nach einem der vorangehenden Ansprüche, welcher folgendes enthält: einen elastischen Beutelteil (13), der den Schwingungserzeuger (3) umgibt, wobei der Innenraum des Beutelteils (13) beim Zusammenpressen unter Druck gesetzt wird und ein Teil des Beutelteils (13) bei Betätigung durch den Schwingungserzeuger (3) als Schwingteil (4) wirkt.

8. Vorrichtung nach Anspruch 7, bei welcher der Beutelteil (13) die Form eines Kissens aufweist.

9. Vorrichtung nach Anspruch 7, bei welcher der Beutelteil (13) die Form einer Matratze aufweist.

10. Vorrichtung nach einem der vorangehenden Ansprüche, welche zusätzlich eine Druckeinrichtung (7) enthält.

11. Vorrichtung nach einem der vorangehenden Ansprüche, wobei der unter Druck stehende geschlossene Gasraum (10) mit Luft gefüllt ist.

FIG.1

FIG.2

EP 0 251 430 B1

FIG.3